(19) Europäisches Patentamt
European Patent Office
Office européen des brevets

(11) Publication number: 0 459 829 A1

# (12) EUROPEAN PATENT APPLICATION

(21) Application number: 91304962.3

(51) Int. Cl.[5]: C07D 253/06, A61K 31/53

(22) Date of filing: 31.05.91

(30) Priority: 01.06.90 GB 9012312

(43) Date of publication of application: 04.12.91 Bulletin 91/49

(84) Designated Contracting States: AT BE CH DE DK ES FR GB GR IT LI LU NL SE

(71) Applicant: THE WELLCOME FOUNDATION LIMITED
Unicorn House 160 Euston Road
London NW1 2BP (GB)

(72) Inventor: Leach, Michael John, The Wellcome Research Lab.
Langley Court
Beckenham, Kent BR3 3BS (GB)
Inventor: Nobbs, Malcolm Stuart, The Wellcome Research Lab.
Langley Court
Beckenham, Kent BR3 3BS (GB)

(74) Representative: Stott, Michael John et al
The Wellcome Research Laboratories Group
Patents & Agreements Langley Court
Beckenham Kent BR3 3BS (GB)

## (54) Pharmacologically active CNS compounds.

(57) The invention provides compounds of formula (I)

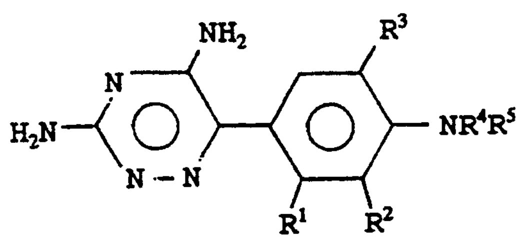

wherein at least one of $R^1$, $R^2$ and $R^3$ is chloro and the others are selected from hydrogen and chloro; and $R^4$ and $R^5$ are the same or different and are each alkyl or hydrogen.

The compounds may be used for the treatment or prophylaxis of a neurodegenerative of other neurological disorder of the CNS, the aetiology of which includes excessive release of the neurotransmitter glutamate.

EP 0 459 829 A1

The present invention relates to phenyl triazines to methods for their manufacture, to pharmaceutical formulations containing them and to their use in therapy, in particular the treatment of a range of central nervous system (CNS) disorders and disease states.

Glutamate is an excitatory amino acid which functions as a neurotransmitter. However, when its extracellular concentration is sufficiently high, glutamate acts as a powerful neurotoxin, capable of killing neurones in the central nervous system, (Rothman & Olney (1986) Prog.Brain.Res., 63, 69). The neurotoxic effect of glutamate has been implicated in a number of central nervous system disorders and disease states including cerebral ischaemic damage and chronic neurodegenerative disorders, such as Alzheimer's disease, motor system disorders, and Huntington's chorea, (Meldrum Clinical Science (1985) 68:113-122). In addition, glutamate has been implicated in other neurological disorders such as epilepsy, manic depression, depression, schizophrenia, high pressure neurological syndrome, chronic pain, trigeminal neuralgia and migraine.

In EP-A-021121 there is disclosed a group of 3,5-diamino-6-(substituted phenyl)-1,2,4-triazines which are active in the treatment of CNS disorders, for example in the treatment of epilepsy. One compound described in that application, 3,5-diamino-6-(2,3-dichlorophenyl)-1,2,4-triazine (lamotrigine), has since been shown to inhibit the release of the excitatory amino acids, glutamate and aspartate, (Leach et al, Epilepsia 27 490-497 1986, A.A. Miller et al a New anticonvulsant drugs. Ed. Meldrum and Porter 165-177, 1987).

A novel class of phenyl triazines has now been found that are potent inhibitors of extracellular glutamate release and are additionally relatively ineffective in inhibiting the enzyme dihydrofolate reductase (DHFR).

Accordingly, the present invention provides a compound of formula (I) or salt thereof

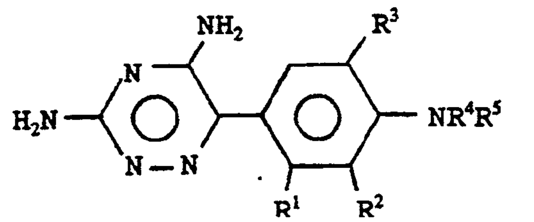

(I)

wherein at least one of $R^1$, $R^2$ and $R^3$ is chloro, and the others are selected from hydrogen and chloro; and $R^4$, $R^5$ are the same or different and are each alkyl or hydrogen.

Preferably $R^4$ and $R^5$ both represent hydrogen. Preferred alkyl moieties are those with 1 to 6 carbon atoms, more preferably 1 to 4 carbon atoms and most preferably methyl or ethyl.

Preferred compounds and their salts according to the invention are:

3,5-Diamino-6-(4-amino-2,3,5-trichlorophenyl)-1,2,4-triazine.

3,5-Diamino-6-(4-amino-2,3-dichlorophenyl)-1,2,4-triazine.

3,5-Diamino-6-(4-amino-3,5-dichlorophenyl)-1,2,4-triazine, and

3,5-Diamino-6-(4-amino-2,5-dichlorophenyl)-1,2,4-triazine.

The present invention also provides the first practicable medical use of the compounds of the present invention.

Compounds of Formula (I) may be used in the treatment or prophylaxis in a mammal of a neurodegenerative or other neurological disorder of the central nervous system, the aetiology of which includes excessive release of the neurotransmitter glutamate. Such conditions can be either acute or chronic. Acute conditions include cerebral ischaemic damage which may arise from a variety of causes including stroke, cardiac arrest, cardiac bypass surgery, neonatal anoxia and hypoglycaemia; and also physical injury or trauma of the spinal cord or brain. Chronic disorders include Alzheimer's disease, Huntington's chorea, Olivopontocerebrellar atrophy and motor system disorders. Other neurological conditions which may be treated with a compound of Formula (I) include depression, manic depression, schizophrenia, chronic pain, epilepsy, trigeminal neuralgia and migraine.

According to a further aspect, the present invention provides a method for the treatment or prophylaxis in a mammal, including man, of a neurodegenerative or other neurological disorder of the central nervous system, the aetiology of which includes excessive release of the neurotransmitter glutamate which comprises administering to the said mammal a non-toxic effective amount of a compound of Formula (I) or salt thereof.

In particular, the invention provided a method of treating a mammal predisposed to or having increased extracellular glutamate levels of the CNS comprising the administration to the said mammal of a non-toxic effective amount of a compound of formula (I) or salt thereof.

The invention also provides a compound of formula (I) or a salt thereof for use in therapy, in particular the treatment or prophylaxis of a neurodegenerative or other neurological disorder of the central nervous system, the aetiology of which includes excessive release of the neurotransmitter glutamate. The invention further provides the use of a compound of formula (I) or a salt thereof for the manufacture of a medicament for the treatment or prophylaxis of a neurodegenerative or other neurological disorder of the central nervous system, the aetiology of which includes excessive release of the neurotransmitter glutamate.

The present invention includes compounds of formula (I) in the form of salts, in particular acid addition salts. Suitable salts include those formed with both organic or inorganic acids. Such acid addition salts will normally be pharmaceutically acceptable although salts of non-pharmaceutically acceptable acids may be of utility in the preparation and purification of the compound in question. Thus, preferred salts include those formed from hydrochloric, hydrobromic, sulphuric, citric, tartaric, phosphoric, lactic, pyruvic, acetic, succinic, oxalic, fumaric, maleic, oxaloacetic, methanesulphonic, ethanesulphonic, p-toluenesulphonic, benzenesulphonic and isethionic acids. Salts of the compounds of formula (I) can be made by reacting the appropriate compound in the form of the free base with the appropriate acid.

While it is possible for the compounds of Formula (I) to be administered as the raw chemical, it is preferable to present them as a pharmaceutical formulation. The formulations of the present invention comprise a compound of Formula (I), as above defined, or a pharmaceutically acceptable salt thereof, together with one or more pharmaceutically acceptable carriers therefor and optionally other therapeutic ingredients. The carrier(s) must be 'acceptable' in the sense of being compatible with the other ingredients of the formulation and not deleterious to the recipient thereof.

The formulations include those suitable for oral, parenteral (including subcutaneous, intradermal, intramuscular and intravenous), rectal and topical (including dermal, buccal and sublingual) administration although the most suitable route may depend upon for example the condition and disorder of the recipient. The formulations may conveniently be presented in unit dosage form and may be prepared by any of the methods well known in the art of pharmacy. All methods include the step of bringing into association a compound of Formula (I) or a pharmaceutically acceptable salt thereof ("active ingredient") with the carrier which constitutes one or more accessory ingredients. In general the formulations are prepared by uniformly and intimately bringing into association the active ingredient with liquid carriers or finely divided solid carriers or both and then, if necessary, shaping the product into the desired formulation.

Formulations of the present invention suitable for oral administration may be presented as discrete units such as capsules, cachets or tablets each containing a predetermined amount of the active ingredient; as a powder or granules; as a solution or a suspension in an aqueous liquid or a non-aqueous liquid; or as an oil-in-water liquid emulsion or a water-in-oil liquid emulsion. The active ingredient may also be presented as a bolus, electuary or paste.

A tablet may be made by compression or moulding, optionally with one or more accessory ingredients. Compressed tablets may be prepared by compressing in a suitable machine the active ingredient in a free-flowing form such as a powder or granules, optionally mixed with a binder, lubricant, inert diluent, lubricating, surface active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein.

Formulations for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilised) condition requiring only the addition of the sterile liquid carrier, for example, water-for-injection, immediately prior to use. Extemporaneous injection solutions and suspensions may be prepared from sterile powders, granules and tablets of the kind previously described.

Formulations for rectal administration may be presented as a suppository with the usual carriers such as cocoa butter or polyethylene glycol.

Formulations for topical administration in the mouth, for example buccally or sublingually, include lozenges comprising the active ingredient in a flavoured basis such as sucrose and acacia or tragacanth, and pastilles comprising the active ingredient in a basis such as gelatin and glycerin or sucrose and acacia.

Preferred unit dosage formulations are those containing an effective dose, an hereinbelow recited, or an appropriate fraction thereof, of the active ingredient.

It should be understood that in addition to the ingredients particularly mentioned above, the formulations of this invention may include other agents conventional in the art having regard to the type of formulation in question, for example those suitable for oral administration may include flavouring agents.

Tablets or other forms of presentation in discrete units may conveniently contain an amount of compound of the Formula (I) which is effective at such dosage or as a multiple of the same, for instances, units containing 5mg to 500mg, usually around 10mg to 250mg.

The compounds of the Formula I are preferably used to treat CNS disorders or diseases by oral administration or injection (intraparenteral or subcutaneous). The precise amount of compound administered to a patient will be the responsibility of the attendant physician. However the dose employed will depend on a number of factors, including the age and sex of the patient, the precise disorder being treated, and its severity. Thus for example when treating a patient with epilepsy the dose range is likely to be significantly lower than when treating a patient after stroke to alleviate cerebral ischaemic damage. Also the route of administration is likely to vary depending on the condition and its severity.

The compounds of the Formula (I) may be administered orally or via injection at a dose of from 0.1 to 30mg/kg per day. The dose range for adult humans is generally from 8 to 2,400 mg/day and preferably 35 to 1,050 mg/day. It may be advantageous to administer an initial dose of 70 to 2,400 mg the first day then a lower dose of 20 to 1,200 mg on subsequent days.

Compounds of Formula (I) may be made by any methods known in the art for related compounds, see for example EP-A-021 121.

Compounds according to the invention in which $R^4$ and $R^5$ are both hydrogen can be prepared by reducing a compound of formula (II)

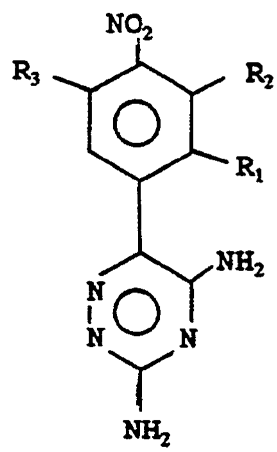

(II)

wherein $R^1$, $R^2$ and $R^3$ are as defined above. The compound of Formula (I) in which $R^4$ and $R^5$ are both hydrogen may then optionally be converted into a compound of Formula (I) in which $R^4$ and/or $R^5$ is alkyl.

The reaction can be carried out with a suitable reducing agent for example utilising platinium oxide as catalyst and hydrogen. The reaction may be carried out in a suitable solvent such as an acidified alkanolic solvent for example acetic acid and methanol.

Compounds of Formula (II) are novel and are, therefore, a further aspect of the present invention. They can be made from compounds known in the art (see EP-A-021 121), for example by reacting a compound of formula (III)

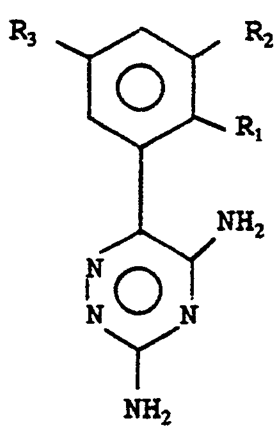

(III)

with potassium nitrate in concentration sulphuric acid.

Compounds according to the invention in which $R^4$ and/or $R^5$ is alkyl may be made from the corresponding compound in which $R^4$ and $R^5$ are hydrogen by a process of reductive alkylation or by reduction of the corresponding amide, which may itself be prepared by reaction of the compound in which $R^4$ and $R^5$ are both hyd-

rogen with an acid halide.

The following examples serve to illustrate the present invention.

Example 1

3,5-Diamino-6-(4-amino-2,3,5-trichlorophenyl)-1,2,4-triazine

(i) 2,3,5,-Trichlorobenzoic acid

Powdered sodium nitrate (15.75g, 0.23M) was added portionwise to concentrated sulphuric acid (113.95ml) which was stirred under an atmosphere of nitrogen. The temperature of the mixture was not allowed to rise above 10°C. 3-Amino-2,5-dichlorobenzoic acid (42.68g, 0.21M) (Sigma) was dissolved in hot glacial acetic acid (518ml), cooled rapidly to room temperature and added dropwise to the above, stirred and cooled so that the internal temperature did not rise above 10°C. The resulting solution was left stirring at room temperature for 2 hours and was then slowly added to a stirred solution of cuprous chloride (41.44g, 0.42M) in concentrated hydrochloride acid (414.40ml). The resultant mixture was stirred at room-temperature for 21 hours. The solid was filtered off, washed well with water, sucked dry, dissolved in ethylacetate, charcoaled, dried over anhydrous magnesium sulphate, filtered and the filtrate evaporated down under reduced-pressure. The solid was dried in vacuo, yield 34.35g m.p. 153-155°C.

(ii) 2,3,5-Trichlolrobenzoyl chloride

A mixture of 2,3,5-trichlorobenzoic acid (5g, 0.02M) and dry toluene (40ml) in thionyl chloride (13.45ml) was refluxed for 3 hours. The cooled solution was evaporated down in vacuo and the residue distilled under nitrogen. Yield 4.50g, b.p. 90°C at 0.70mmHg.

(iii)2,3,5-Trichlorobenzoyl cyanide

A mixture of cuprous cyanide (4.35g, 0.05M), potassium iodide (7.37g, 0.04M) and xylene (40.35ml) was refluxed in an atmosphere of nitrogen under a Dean and Stark trap for 24 hours. A solution of 2,3,5-trichlorobenzoyl chloride (4.40g, 0.02M) in sodium dried xylene (10ml) was added to the above suspension and the mixture refluxed for 3 days. The resulting mixture was refluxed for 3 days, filtered and the solid washed well with sodium dried xylene (30ml). The filtrate and washings were combined and evaporated in vacuo to give an oil. Yield 4.43g (quantitative).

(iv) 3,5-Diamino-6-(2,3,5-trichlorophenyl)-1,2,4-triazine

A solution of 2,3,5-trichlorobenzoyl cyanide (30.55g, 0.13M) was dissolved in acetonitrile (100ml) and added dropwise to a suspension of aminoguanidine bicarbonate (35g, 0.26M) in dilute sulphuric acid (150 ml concentrated $H_2SO_4$ / 150 ml $H_2O$). The temperature was maintained below 30°C. The mixture was stirred at room temperature for 3 days. The solid was filtered, washed with water (2x100ml) and sucked dry. A suspension of the solid in a 10% solution of sodium hydroxide pellets in water (100ml) was stirred at room temperature for 1 hour. The solid was filtered, washed with water and dried in vacuo. The solid was refluxed with hot n-propanol for 1.5 hours, filtered and dried in vacuo at 80°C. Yield 5.64g, mp. 225-227°C.

Solvent DMSO-$d_6$

NMR Assigment 6.40 δ (s,2H,-$NH_2$), 6.70 δ (br.s, 2H,-$NH_2$), 7.40 δ (s,1H), 7.80 δ (s,1H).

(v) 3,5-Diamino-6-(2,3,5-trichloro-4-notrophenyl)-1,2,4-triazine

To a solution of 3,5-diamino-6-(2,3,5-trichlorophenyl)-1,2,4-triazine (1.05g, 0.004M) in concentrated sulphuric acid (17.85ml) was added potassium nitrate (0.56g, 0.006M). After stirring at room temperature for 10 hours, the solution was poured onto ice and basified with 10N NaOH. The product was extracted with ethylacetate, bulked, dried ($MgSO_4$) and evaporated. Chromatography on $SiO_2$ eluting with ethyl acetate gave the desired product, 0.347g, m.p. 258-260°C.

Solvent DMSO-$d_6$

NMR Assignment 6.60 δ (s,2H,-$NH_2$), 6.90 δ (br.s,2H,-$NH_2$), 8.40 δ (s,1H).

(vi) 3,5,-Diamino-6-(4-amino-2,3,5-trichlorophenyl)-1,2,4- triazine

A solution of 3,5-diamino-6-(2,3,5-trichloro-4-nitrophenyl)-1,2,4-triazine (0.25g, 0.0007M) in acetic acid (12ml) / methanol (1ml) was reduced under an atmosphere of hydrogen in the presence of $PtO_2$ (0.12g). The mixture was filtered and the filtrate was concentrated. The residue was neutralised with saturated $NaHCO_3$ solution and the product was extracted with ethylacetate, bulked, dried ($MgSO_4$) and evaporated, 0.045g m.p. 225-226°C.
Solvent DMSO-$d_6$
NMR Assignment 5.20 δ (br.s,2H,-$NH_2$), 6.30 δ (br.s,2H,-$NH_2$), 6.50 δ (br.s,2H,-$NH_2$), 7.60 δ (s,1H).

Example 2

3,5-Diamino-6-(4-amino-3,5-dichlorophenyl)-1,2,4-triazine

(i) 3,5-Dichloro-4-dimethylamino-methyleneaminobenzoylchloride

Dimethylformamide (4.20ml) was added to a suspension of 4-amino-3,5-dichlorobenzoic acid (10.00g. 0.049M, Lancaster Synthesis) in thionyl chloride (134.60ml) and the resultant solution was refluxed for 5 hrs. The cooled solution was evaporated down in vacuo and the residue azeotroped with dry toluene (4x50ml). Yield - 13.50g (quantitative).

(ii) 3,5-Dichloro-4-dimethylamino-methyleneaminobenzolycyanide

A mixture of cuprous cyanide (7.98g. 0.089M), potassium iodide (13.51g. 0.081M) and xylene (100ml) was refluxed in an atmosphere of nitrogen under a Dean and Stark trap for 24 hrs. A suspension of 3,5-dichloro-4-dimethylamino-methyleneaminobenzoylchloride (13.40g. 0.048M) in sodium dried xylene (30ml) was added to the above suspension. The resulting mixture was refluxed for 3 days, filtered and the solid was washed well with sodium dried xylene (50ml). The filtrate and washings were combined and evaporated in vacuo to give an oil.
Yield = 13.35g (quantitative).

(iii) 3.5-Diamino-6-(3,5-dichloro-4-dimethylamino-methyleneaminophenyl)-1,2,4-triazine

A suspension of 3,5-dichloro-4-dimethylamino-methyleneaminobenzoylcyanide (13.35g, 0.049M) was dissolved in acetonitrile (39ml) and added dropwise to a suspension of aminoguanidine bicarbonate (13.31g. 0.098M) in dilute sulphuric acid (57ml concentrated $H_2SO_4$/57ml$H_2O$). The temperature was maintained below 30°C. The mixture was stirred at room temperature for 3 days. The solid was filtered and washed with water (2x50ml). The filtrate was basified with 10N Sodium hydroxide (50ml) and extracted with ethylacetate, bulked, dried ($MgSO_4$) and evaporated. The solid was refluxed with n-propanol for 1.5 hrs and evaporated. Chromatography on $SiO_2$ gel, elutint with chloroform to 6% methanol, gave the desired product as a yellow solid.

(iv) 3,5-Diamino-6-(4-amino-3,5-dichlorophenyl)-1,2,4-triazine

A suspension of 3,5-diamino-6-(3,5-dichloro-4-dimethylamino-methyleneaminophenyl)-1,2,4-triazine (0.20g, 0.00061M) in absolute ethanol (5ml) and 0.880 ammonia (6ml) was stirred and heated in a bomb at 110°C for 48 hrs. The mixture was cooled and evaporated. Chromotography on $SiO_2$ gel eluting with chloroform: methanol, 98:2 to 95:5 gave the desired product, 0.065g. m.p. 253-256°C.
Solvent DMSO-$d_6$
NMR Assignment 5.60 δ (br, s, 2H, -NH2), 6.20 δ (br, s, 2H,-NH2), 6.60 δ (br, s, 2H, -NH2), 7.30 δ (s, 2H).

Pharmacological Activity

Inhibition of Glutamate release and Inhibition of Rat Liver DHRF

A compound of Formula (I) was tested for its effect on veratrine-evoked release of glutamate from rat brain slices according to the protocol described in Epilepsia 27(5): 490-497, 1986. The protocol for testing for inhibition of DHFR activity was a modification of that set out in Biochemical Pharmacology Vol.20 pp 561-574, 1971.

The results are given in Table 1, the $IC_{50}$ being the concentration of compound to cause 50% inhibition of

(a) veratrine-evoked release of glutamate and (b) of DHFR enzyme activity.

**TABLE I**

| Compound of Example No. | $IC_{50}$ ($\mu M$) Glutamate Release (P95 limits) | $IC_{50}$ ($\mu M$) Rat Liver DHFR (P95 limits) |
|---|---|---|
| 1 | < 10 | > 100 |

Pharmaceutical Formulation Example

A: Tablets

| | |
|---|---|
| Compound of Example 1 | 150 mg ) |
| Lactose | 200 mg ) |
| Maize Starch | 50 mg ) |
| Polyvinylpyrrolidone | 4 mg ) |
| Magnesium Stearate | 4 mg ) |

) = contents per tablet.

The drug was mixed with lactose and starch and granulated with a solution of the polyvinylpyrrolidone in water. The resultant granules were dried, mixed with magnesium stearate and compressed to give tablets.

B: Injections

Injection I

The salt of the compound of formula I was dissolved in sterile water for injection.

Intravenous Injection Formulation II

| | |
|---|---|
| Active ingredient | 0.20g |
| Sterile, pyrogen-free phosphate buffer (pH9.0) to | 10ml |

The compound of example 1 as a salt is dissolved in mont of the phosphate buffer at 35-40°C, then made up to volume and filtered through a sterile micropore filter into sterile 10ml glass vials (Type 1) which are sealed with sterile closures and overseals.

In the following Examples, the active compound maybe any compound of formula (I) or a pharmaceutically acceptable salt thereof.

C: Capsule formulations

Capsule Formulation I

Formulation I may be prepared by admixing the ingredients and filling two-part hard gelatin capsules with the resulting mixture.

| | | mg/capsule |
|---|---|---|
| (a) | Active ingredient | 250 |
| (b) | Lactose B.P. | 143 |
| (c) | Sodium Starch Glycollate | 25 |
| (d) | Magnesium Stearate | 2 |
| | | ---- |
| | | 420 |

Capsule Formulation II

| | | mg/capsule |
|---|---|---|
| (a) | Active ingredient | 250 |
| (b) | Macrogel 4000 BP | 350 |
| | | ----- |
| | | 600 |

Capsules may be prepared by melting the Macrogel 4000 BP, dispersing the active ingredient in the melt, and filling two-part hard gelatin capsules therewith.

Capsule Formulation III (Controlled release capsule)

| | | mg/capsule |
|---|---|---|
| (a) | Active ingredient | 250 |
| (b) | Microcrystalline Cellulose | 125 |
| (c) | Lactose BP | 125 |
| (d) | Ethyl Cellulose | 13 |
| | | ----- |
| | | 513 |

The controlled-release capsule formulation may be prepared by extruding mixed ingredients (a) to (c) using an extruder, then spheronising and drying the extrudate. The dried pellets are coated with ethyl cellulose (d) as a controlled-release membrane and filled into two-part hard gelatin capsules.

D: Syrup formulation

| | | |
|---|---|---|
| Active ingredient | | 0.2500 g |
| Sorbitol Solution | | 1.5000 g |
| Glycerol | | 1.0000 g |
| Sodium Benzoate | | 0.0050 g |
| Flavour | | 0.0125 ml |
| Purified water | q.s. to | 5.0 ml |

The sodium benzoate is dissolved in a portion of the purified water and the sorbitol solution added. The active ingredient is added and dissolved. The resulting solution is mixed with the glycerol and then made up to the required volume with the purified water.

E: Suppository Formulation

mg/suppository

| | |
|---|---|
| Active ingredient (63$\mu$m)* | 250 |
| Hard Fat, BP (Witepsol H15 - Dynamit Nobel | 1170 |
| | ----- |
| | 2020 |

* The active ingredient is used as a powder wherein at least 90% of the particles are of 63$\mu$m diameter or less.

One-fifth of the Witepsol H15 is melted in a steam-jacketed pan at 45°C maximum. The active ingredient is sifted through a 200µm sieve and added to the molten base with mixing, using a Silverson fitted with a cutting head, until a smooth dispersion is achieved. Maintaining the mixture at 45°C, the remaining Witepsol H15 is added to the suspension which is stirred to ensure a homogenous mix. The entire suspension is then passed through a 250µm stainless steel screen and, with continuous stirring, allowed to cool to 40°C. At a temperature of 38-40°C, 2.02g aliquots of the mixture are filled into suitable plastic moulds and the suppositories allowed to cool to room temperature.

## Claims

1. A compound of formula (I) or a salt thereof

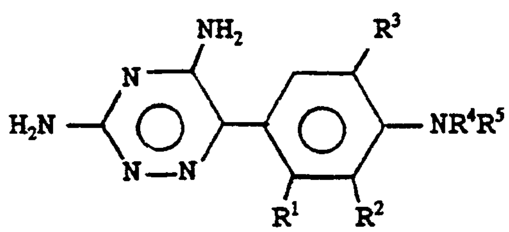

(I)

wherein at least one of $R^1$, $R^2$ and $R^3$ is chloro and the others are selected from hydrogen and chloro; and $R^4$ and $R^5$ are the same or different and are each alkyl or hydrogen.

2. A compound as claimed in claim 1 wherein $R^4$ and $R^5$ both represent hydrogen

3. 3,5-Diamino-6-(4-amino-2,3,5-trichlorophenyl)-1,2,4-triazine or a salt thereof

4. 3,5-Diamino-6-(4-amino-2,3-dichlorophenyl)-1,2,4-triazine;
3,5-diamino-6-(4-amino-3,5-dichlorophenyl)-1,2,4-triazine;
3,5-diamino-6-(4-amino-2,5-dichlorophenyl)-1,2,4-triazine;
or a salt thereof

5. A process for the preparation of a compound of formula (I) as claimed in any of claims 1 to 4 in which $R^4$ and $R^5$ are both hydrogen which comprises reducing a compound of formula (II)

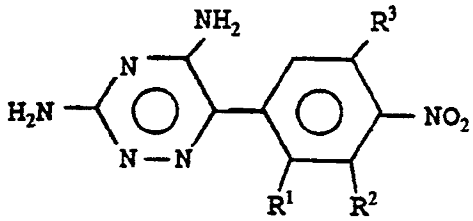

(II)

optionally converting the compound of formula (I) produced in which $R^4$ and $R^5$ are both hydrogen into a compound of formula (I) in which $R^4$ and/or $R^5$ is alkyl; and optionally converting the compound of formula (I) produced in the form of the free base into a salt.

6. A pharmaceutical composition which comprises a compound as claimed in any of claims 1 to 6 or a pharmaceutically acceptable salt thereof together with at least one pharmaceutically acceptable carrier.

7. A compound of formula (II)

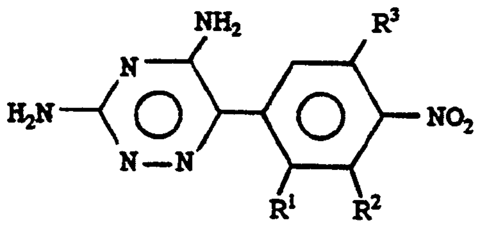

(II)

wherein at least one of $R^1$, $R^2$ and $R^3$ is chloro and the others are selected from hydrogen and chloro.

8. A compound of formula (I) as claimed in any of claims 1 to 4 for use in therapy.

9. A compound of formula (I) as claimed in any of claims 1 to 4 for use in the therapy or prophylaxis of a neurodegenerative or other neurological disorder of the central nervous system, the aetiology of which includes excessive release of the neurotransmitter glutamate.

**Claims for the following Contractings States: ES, GR**

1. A process for the preparation of a compound of formula (I) or a salt thereof

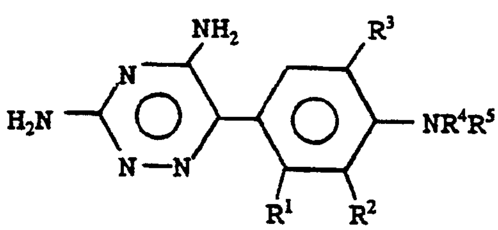

(I)

wherein at least one of $R^1$, $R^2$ and $R^3$ is chloro and the others are selected from hydrogen and chloro; and $R^4$ and $R^5$ are the same or different and are each alkyl or hydrogen;
which comprises reducing a compound of formula (II)

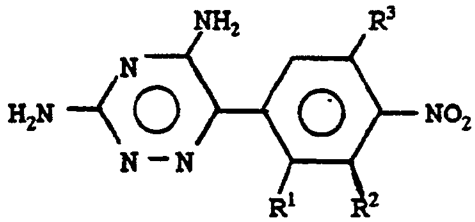

(II)

optionally converting the compound of formula (I) produced in which $R^4$ and $R^5$ are both hydrogen into a compound of formula (I) in which $R^4$ and/or $R^5$ is alkyl; and
optionally converting the compound of formula (I) produced in the form of the free base into a salt.

2. A process as claimed in claim 1 for the production of compounds wherein $R^4$ and $R^5$ both represent hydrogen.

3. A process as claimed in claim 1 for the production of 3,5-diamino-6-(4-amino-2,3,5-trichlorophenyl)-1,2,4-triazine or a salt thereof.

4. A process as claimed in claim 1 for the production of
   3,5-diamino-6-(4-amino-2,3-dichlorophenyl)-1,2,4-triazine;
   3,5-diamino-6-(4-amino-3,5-dichlorophenyl)-1,2,4-triazine;
   3,5-diamino-6-(4-amino-2,5-dichlorophenyl)-1,2,4-triazine;
   or a salt thereof.

European Patent Office

# EUROPEAN SEARCH REPORT

Application Number

EP 91304962.3

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,A | EP - A1 - 0 021 121 (WELLCOME) * Claims 1,4 * -- | 1,6-9 | C 07 D 253/06<br>A 61 K 31/53 |
| A | EP - A1 - 0 021 120 (WELLCOME) * Claims 1,10 * -- | 1,6-9 | |
| A | EP - A1 - 0 247 892 (WELLCOME) * Claims 1,6-9 * -- | 1,6-9 | |
| | EP - A2 - 0 142 306 (WELLCOME) * Claims 1,8 * -- | 1,6,7 | |
| A | US - A - 3 637 688 (REES) * Claim 1 * -- | 1 | |
| A | CHEMICAL ABSTRACTS, vol. 98, no. 11, March 14, 1983, Columbus, Ohio, USA BAXTER, MARTIN G. et al. "Substituted aromatic compounds." page 563, column 2, abstract-no. 89 397d ---- | 1,6,8, 9 | TECHNICAL FIELDS SEARCHED (Int. Cl.5)<br>C 07 D 253/00 |

The present search report has been drawn up for all claims

| Place of search | Date of completion of the search | Examiner |
|---|---|---|
| VIENNA | 02-08-1991 | HAMMER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)